# EUROPÄISCHE PATENTSCHRIFT

(11) **EP 1 050 219 B1**
(45) Veröffentlichungstag und Bekanntmachung des Hinweises auf die Patenterteilung: **20.11.2002**
(21) Anmeldenummer: 00108826.9
(22) Anmeldetag: 26.04.2000
(51) Int. Cl.: A23K 1/16

(54) **D-Pantothensäure und/oder eines ihrer Salze enthaltende Futtermittel-Additive und Verfahren zu deren Herstellung**
Feed additive containing D-pantothenic acid and/or one of its salts and method for the preparation thereof
Additif alimentaire pour animaux contenant de l'acide D-pantothénique et/ou un de ses sels et procédé de fabrication correspondant

(30) Priorität: 05.05.1999 DE 19920507; 31.03.2000 DE 10016321
(43) Veröffentlichungstag der Anmeldung: 08.11.2000
(73) Patentinhaber: Degussa AG, 40474 Düsseldorf (DE)
(72) Erfinder: Binder, Michael, Dr., 33803 Steinhagen (DE); Uffmann, Klaus-Erich, 33611 Bielefeld (DE); Walger, Ilona, Dr., 33607 Bielefeld (DE); Becker, Ulrich, Dr., 97611 Selce (SK); Pfefferle, Walter, Dr., 33790 Halle (DE); Friedrich, Heinz, Dr., 63457 Hanau (DE)

(56) Entgegenhaltungen:
- GB-A- 598 177
- GB-A- 784 434
- EGGELING, L., MORBACH, S., AND SAHM, H.: "The fruits of molecular physiology: engineering the L-isoleucine biosynthesis pathway in Corynebacterium glutamicum" JOURNAL OF BIOTECHNOLOGY., Bd. 56, 1997, Seiten 167-182, XP004103426 ELSEVIER SCIENCE PUBLISHERS, AMSTERDAM., NL ISSN: 0168-1656

## Beschreibung

Die Erfindung betrifft ein Tierfuttermittel-Additiv auf Fermentationsbrühebasis, das D-Pantothensäure und / oder eines ihrer Salze enthält und Verfahren zur Herstellung dieses Additivs.

### Stand der Technik

Pantothensäure wird weltweit in einer Grössenordnung von mehreren tausend Tonnen pro Jahr produziert. Ein grosser Teil der produzierten Pantothensäure wird für die Ernährung von Nutztieren wie Geflügel und Schweine verwendet. Der Bedarf steigt.

Pantothensäure kann durch chemische Synthese oder biotechnisch durch Fermentation geeigneter Mikroorganismen in geeigneten Nährlösungen hergestellt werden. Bei der chemischen Synthese ist das DL-Pantolacton eine wichtige Vorstufe. Es wird in einem mehrstufigen Verfahren aus Formaldehyd, Isobutylaldehyd und Cyanid hergestellt, in weiteren Verfahrensschritten das racemische Gemisch aufgetrennt, das D-Pantolacton mit β-Alanin kondensiert und so D-Pantothensäure erhalten.

Die typische Handelsform ist das Calcium-Salz der D-Pantothensäure. Das Calcium-Salz des racemischen Gemisches der D,L-Pantothensäure ist ebenfalls gebräuchlich.

Der Vorteil der fermentativen Herstellung durch Mikroorganismen liegt in der direkten Bildung der gewünschten stereoisomeren Form, nämlich der D-Form, die frei von L-Pantothensäure ist.

Verschiedene Arten von Bakterien, wie z. B. Escherichia coli, Arthrobacter ureafaciens, Corynebacterium erythrogenes, Brevibacterium ammoniagenes und auch Hefen, wie z. B. Debaromyces castellii können, wie in EP-A-0 493 060, EP-A-0 590 857 und WO 97/10340 gezeigt, unter geeigneten Fermentationsbedingungen D-Pantothensäure produzieren. Besonders geeignete Mikroorganismen sind die dort beschriebenen Derivate von Escherichia coli IFO3547 wie z. B. die Stämme FV5069/pFV31 oder FV5069/pFV202.

Bei der fermentativen Herstellung der D-Pantothensäure, wie sie in EP-A-0 493 060, EP-A-0 590 857 und WO 97/10340 beschrieben ist, wird ein zur D-Pantothensäure Produktion befähigter Mikroorganismus in einem geeignetem Nährmedium kultiviert und die gebildete D-Pantothensäure anschließend in aufwendiger Weise isoliert, gereinigt und als Calciumsalz dargestellt.

Geeignete Nährmedien enthalten eine Kohlenstoffquelle wie z. B. Glucose oder Stärkemehlhydrolysat oder Sucrose oder Melasse, Vorstufen wie z. B. β-Alanin, D,L-Pantoinsäure oder D,L-Pantolacton, eine Stickstoffquelle wie z. B. Ammoniumsulfat, eine Phosphor-Quelle wie z. B. Kaliumphosphat und weitere Salze, Spurenelemente und Vitamine und gegebenenfalls komplexe Medienzusätze wie z. B. Hefeextrakt. Die Mikroorganismen werden dann in diesem Medium bei einem geeignetem pH-Wert unter entsprechender Belüftung und Rührung inkubiert, wobei diese dann D-Pantothensäure ausscheiden.

Nach dem derzeitigen Stand der Technik, der in WO96/33283 und EP-A-0 590857 dargestellt ist, wird das Calciumsalz der D-Pantothensäure durch eine aufwendige Isolierung und Reinigung aus der pantothensäurehaltigen Fermentationsbrühe gewonnen. Nach einer ersten Abtrennung der Biomasse durch Filtration oder Zentrifugation erfolgt die weitere Aufarbeitung des Filtrates durch Reinigung mittels Aktivkohle oder durch Säulenchromatographie. Nach der Umsetzung der so erhaltenen Lösungen mit Calciumhydroxid läßt man das gewünschte Ca-Salz auskristallisieren.

Gemäß der WO 96/33283 entfärbt man das Filtrat mit Aktivkohle in der ersten Säule. Mit konzentrierter Salzsäure wird ein pH-Wert von 3,0 eingestellt und die Flüssigkeit anschließend kontinuierlich über zwei weitere mit Aktivkohle gepackten Säulen gereinigt. Die Elution der D-Pantothensäure erfolgt mit Hilfe von Methylalkohol. Nach der sich anschließenden Neutralisation mit Ca(OH)₂-Pulver erhält man eine Lösung, aus der man das Calcium D-Pantothenat durch Kristallisation bei 5°C gewinnt.

Bei der in EP-A-0 590 857 beschriebenen Methode reinigt man das Filtrat zunächst mit Hilfe von Kationen- und Anionenaustauschersäulen. Die Elution erfolgt mit Salzsäure. Die eluierte Fraktion wird anschließend mit Ca(OH)₂ neutralisiert, mit Aktivkohle versetzt und abfiltriert. Das gewonnene Filtrat wird dann in einem niedermolekularen Alkohol (Methanol, Ethanol, Isopropanol)extrahiert und das Calcium D-Pantothenat durch Kristallisation gewonnen.

Das auf die beschriebene Weise hergestellte Calcium D-Pantothenat wird als Zusatz in Futtermitteln für die Tierernährung verwendet.

Aus der GB-A-598 177 ist ein Verfahren zur fermentativen Herstellung von 2,3-Butylanglycol bekannt, bei dem dispergierte Maische als Nährboden dient. Als Mikroorganismus wird Aerobacter aerogenes eingesetzt, der in sehr geringen Mengen als Nebenprodukt Pantothensäure produziert.

### Aufgabe der Erfindung

Nach dem Stand der Technik werden Salze der D-Pantothensäure und D,L-Pantothensäure durch Umsetzung der durch chemische Synthese oder Fermentation hergestellten Säuren mit den gewünschten Salzlösungen hergestellt.

Die Aufgabe der Erfindung besteht darin, neue als Futtermitteladditive geeignete Zubereitungsformen der D-Pantothensäure und ihrer Salze zur Verfügung zu stellen.

Weiterhin ist es eine Aufgabe der Erfindung, ein Herstellverfahren zur Verfügung zu stellen, dass ökonomischer und leistungsstärker als die gegenwärtig bekannten Verfahren ist.

### Beschreibung der Erfindung

Gegenstand der Erfindung ist ein Tierfuttermittel-Additiv auf Fermentationsbrühe-Basis, das dadurch gekennzeichnet ist, daß es
a) D-Pantothensäure und/oder eines ihrer Salze, insbesondere Alkali- oder Erdalkali-Salze, in einer Menge von 20 bis 80 Gew.-% (Trockenmasse),
b) die während der Fermentation gebildete Biomasse in einer Menge von 0 bis 100 % und
c) zumindest den überwiegenden Teil der weiteren gelösten Inhaltsstoffe der Fermentationsbrühe enthält und
d) in fester Form, insbesondere feinteilig oder granuliert und rieselfähig, vorliegt.

Die Additive liegen im allgemeinen je nach Anforderung als sprühgetrocknete oder lyophilisierte, feinteilige, rieselfähige Pulver, aber auch in granulierter Form vor, die unterschiedliche Anteile an Biomasse enthalten können. Die Schüttdichte liegt insbesondere bei ca. 500 kg/m³. Die Additive sind lagerstabil.

Wird die Biomasse abgetrennt, werden natürlich auch weitere, zum Beispiel anorganische Feststoffe entfernt. Daneben enthält das erfindungsgemäße Additiv zumindest den überwiegenden Teil der in der Fermentationsbrühe gelöst vorliegenden, weiteren gebildeten oder zugesetzten Stoffe, soweit sie nicht durch geeignete Verfahren abgetrennt wurden.

Zu diesen Stoffen können organische Nebenprodukte gehören, die von den bei der Fermentation eingesetzten Mikroorganismen neben der D-Pantothensäure erzeugt und ausgeschieden werden. Dazu gehören L-Aminosäuren, ausgewählt aus der Gruppe L-Methionin, L-Lysin, L-Valin, L-Threonin, L-Alanin oder L-Tryptophan, insbesondere L-Valin. Dazu gehören weiterhin organische Säuren, die ein bis drei Carboxyl-Gruppen tragen wie z. B. Essigsäure, Milchsäure, Zitronensäure, Äpfelsäure oder Fumarsäure. Schließlich gehören dazu auch schlecht verwertbare Zucker wie z. B. Trehalose. Diese Verbindungen sind gegebenenfalls erwünscht, wenn sie die Wertigkeit des Additivs verbessern.

Zu diesen Stoffen gehören weiterhin Reste der eingesetzten verwertbaren Zucker wie z. B. Glucose oder Saccharose.

Ein weiterer Gegenstand der Erfindung ist ein Verfahren zur Herstellung eines D-Pantothensaüre und/oder eines ihrer Salze in einer Menge von 20 bis 80 Gew.% (Trockenmasse), gekennzeichnet ist, daß man
a) eine im allgemeinen Natrium-, Kalium-, Ammonium-, Magnesium-, oder Calciumsalze der enthaltende D-Pantothensäure-enthaltenden Brühe durch Fermentation herstellt,
b) aus dieser gegebenenfalls vollständig oder teilweise die Biomasse abtrennt,
c) die so erhaltene Lösung bzw. Brühe mit dem Hydroxid oder Oxid eines Erdalkali- oder Alkalimetalls in vorzugsweise stöchiometrischen Mengen, bezogen auf die D-Pantothensäure, versetzt und
d) das so erhaltene Gemisch trocknet, sprühtrocknet, sprühgranuliert oder granuliert.

Ein weiterer Gegenstand der Erfindung ist Verfahren zur Herstellung von Tierfuttermittel-Additiven mit einem Gehalt an D-Pantothensäure und/oder dessen Natrium-, Kalium-, Ammonium-, Magnesium- oder Calciumsalzen in dem Bereich von 20 bis 80 Gew.-% (Trockenmasse) aus Fermentationsbrühen, gekennzeichnet durch die Schritte
a) bevorzugt Entfernen von Wasser aus der Fermentationsbrühe (Aufkonzentration)
b) gegebenenfalls Entfernen der während der Fermentation gebildeten Biomasse in einer Menge von 0 bis 100 %.
c) Zusatz von einer oder mehreren der genannten Verbindungen zu den gemäß a) und b) erhaltenen Fermentationsbrühen, wobei die Menge der zugesetzten Verbindungen so bemessen ist, daß deren Gesamkonzentration im Tierfuttermittel-Additiv im Bereich von etwa 20 bis 80 Gew.-%, insbesondere 50 bis 80 Gew.-%, liegt, und
d) Trocknen der gemäß c) erhaltenen Fermentationsbrühe, um das Tierfuttermittel-Additiv in der gewünschten Pulveroder Granulatform zu erhalten.

Geeignet für das erfindungsgemäße Verfahren sind Fermentationsbrühen, die unter Verwendung von zur Produktion von D-Pantothensäure geeigneten Mikroorganismen gewonnen werden und D-Pantothensäure und/oder deren Salze enthalten.

Bei den Mikroorganismen kann es sich um Pilze oder Hefen wie zum Beispiel Debaromyces castellii oder Gram-positive Bakterien zum Beispiel der Gattung Corynebacterium oder um Gram-negative Bakterien wie zum Beispiel die der Familie Enterobacteriaceae handeln. Bei der Familie der Enterobacteriaceae ist besonders die Gattung Escherichia mit der Art Escherichia coli zu nennen. Innerhalb der Art Escherichia coli sind die sogenannten K-12 Stämme wie zum Beispiel die Stämme MG1655 oder W3110 (Neidhard et al.: Escherichia coli and Salmonella. Cellular and Molecular Biology (ASM Press, Washington D.C.)) oder der Escherichia coli Wildtystamm IFO3547 (Institut für Fermentation, Osaka, Japan) und davon abgeleitete Mutanten zu nennen. Unter den aus aus IFO3547 hergestellten Stämmen zeichnen sich wiederum FV5069/pFV31 (EP-A-0 590 857) und FV5069/pFV202 (WO 97/10340) aus. Bei der Gattung Corynebacterium ist insbesondere die Art Corynebacterium glutamicum zu nennen.

Die oben beschriebenen Mikroorganismen können kontinuierlich oder diskontinuierlich im batch - Verfahren (Satzkultivierung) oder im fed batch (Zulaufverfahren) oder repeated fed batch Verfahren (repetitives Zulaufverfahren> zum Zwecke der D-Pantothensäure-Produktion kultiviert werden. Eine Zusammenfassung über bekannte Kultivierungsmethoden sind im Lehrbuch von Chmiel (Bioprozesstechnik 1. Einführung in die Bioverfahrenstechnik (Gustav Fischer Verlag, Stuttgart, 1991) oder im Lehrbuch von Storhas (Bioreaktoren und periphere Einrichtungen (Vieweg Verlag, Braunschweig/Wiesbaden, 1994) beschrieben.

Das zu verwendende Kulturmedium muß in geeigneter Weise den Ansprüchen der jeweiligen Mikroorganismen genügen. Beschreibungen von Kulturmedien verschiedenener Mikroorganismen sind im Handbuch "Manual of Methods for General Bacteriology" der American Society for Bacteriology (Washington D.C., USA, 1981) enthalten. Als Kohlenstoffquelle können Zucker und Kohlehydrate wie z.B. Glucose, Saccharose, Lactose, Fructose, Maltose, Melasse, Stärke und Cellulose, Öle und Fette wie z. B. Sojaöl, Sonnenblumenöl, Erdnussöl und Kokosfett, Fettsäuren wie z. B. Palmitinsäure, Stearinsäure und Linolsäure, Alkohole wie z. B. Glycerin und Ethanol und organische Säuren wie z. B. Essigsäure verwendet werden. Diese Stoffe können einzeln oder als Mischung verwendet werden. Als Stickstoffquelle können organische Stickstoff haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden. Als Phosphorquelle können Kaliumdihydrogenphosphat oder Dikaliumhydrogenphosphat oder die entsprechenden Natrium haltigen Salze verwendet werden. Das Kulturmedium muß weiterhin Salze von Metallen enthalten wie z.B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies Vorstufen der D-Pantothensäure wie Aspartat, β-Alanin, Ketoisovalerat, Ketopantoinsäure oder Pantoinsäure und gegebenenfalls deren Salze zugesetzt werden. Die genannten Einsatzstoffe können zur Kultur in Form eines einmaligen Ansatzes hinzugegeben oder in geeigneter Weise während der Kultivierung zugefüttert werden.

Zur Kontrolle des pH-Wertes werden bevorzugt Ammoniak oder Ammoniakwasser verwendet.Andere basische Verbindungen wie Natriumhydroxid oder Kaliumhydroxid sind ebenfallsgeeignet. Werden saure Verbindungen benötigt,setzt man Phosphorsäure oder Schwefelsäure in geeigneter Weise ein. Zur Kontrolle der Schaumentwicklung verwendet man Antischaummittel wie z.B. Fettsäurepolyglykolester. Zur Aufrechterhaltung der Stabilität von Plasmiden werden dem Medium gegebenenfalls geeignete selektiv wirkende Stoffe z.B. Antibiotika hinzugefügt. Um aerobe Bedingungen aufrechtzuerhalten, werden Sauerstoff oder Sauerstoff haltige Gasmischungen wie z.B. Luft in die Kultur eingetragen. Die Temperatur der Kultur liegt normalerweise bei 20°C bis 45°C und vorzugsweise bei 25°C bis 40°C. Die Kultur wird solange fortgesetzt, bis sich ein Maximum an D-Pantothensäure gebildet hat. Dieses Ziel wird normalerweise innerhalb von 10 Stunden bis 160 Stunden erreicht.

Die so erhaltenen Fermentationsbrühen haben üblicherweise eine Trockenmasse von 7,5 bis 25 Gew.-% und enthalten D-Pantothensäure in einer Konzentration von > 0 bis 20 Gew.-%. Besonders vorteilhaft sind solche Fermentationsverfahren, bei denen die D-Pantothensäure zu 2 bis 20 Gew.-% in der Trockenmasse nach Beendigung der Fermentation vorliegt. Vorteilhaft ist außerdem auch, wenn die Fermentation zumindest am Ende, vorteilhaft jedoch über mindestens 30 % der Fermentationsdauer zuckerlimitiert gefahren wird. Das heißt, daß während dieser Zeit die Konzentration an verwertbarem Zucker im Fermentationsmedium auf ≥ 0 bis 3 g/l gehalten bzw. darauf abgesenkt wird.

In einer Ammoniumionen enthaltenden Variante zur Herstellung der erfindungsgemäßen Additive werden die D-Pantothensäure-haltigen Fermentationsbrühen, gegebenenfalls zunächst durch bekannte Separationsmethoden wie zum Beispiel der Zentrifugation, der Filtration, dem Dekantieren oder einer Kombination hieraus vollständig oder zum Teil von der Biomasse befreit. Es ist erfindungsgemäß jedoch auch möglich, die Biomasse gänzlich in der Fermentationsbrühe zu belassen. Anschließend wird diese Brühe im allgemeinen mit 0,8 bis 1,2, vorzugsweise 0,95 bis 1,1 Äquivalenten, eines Oxids oder Hydroxids eines Alkalioder Erdalkali-Metalles, insbesondere NaOH, KOH, Ca(OH)₂ oder MgO, bezogen auf die D-Pantothensäure, versetzt. Bei geringen D-Pantothensäurekonzentrationen kann es vorteilhaft sein, auch deutlich höhere Mengen der Oxide oder Hydroxide einzusetzen, zum Beispiel im Bereich 1,2 bis 4 Äquivalenten. Die auf diese Weise erhaltene Suspension wird vor der Trocknung im allgemeinen auf maximal 60 Gew.-% Trockenmasse konzentriert. Es ist gleichfalls möglich, die Fermentationsbrühe zunächst zu konzentrieren und dann die Oxide oder Hydroxide hinzuzufügen. Das erhaltene Konzentrat wird dann in einem üblichen Trockner oder beispielsweise mit Hilfe eines Fallfilmverdampfers oder eines Dünnschichtverdampfers oder eines Sprühtrockners oder eines Sprühgranulators oder einer Gefriertrocknungsanlage als rieselfähiges, frei fließendes, feinteiliges Pulver oder Granulat gewonnen. Eine Granulation kann auch im Anschluß an die Trocknung stattfinden, zum Beispiel in Form der Aufbaugranulation.

Die Erfinder fanden weiterhin eine neue Methode, um Ammonium-, Kalium-, Natrium-, Magnesium- oder Calcium- D-Pantothensäure-Salze enthaltende Pulver oder diese enthaltende Zubereitungsformen auf schnelle und kostengünstige Weise herzustellen. Hierzu wird eine unter Verwendung der entsprechenden Hydroxy-Verbindungen hergestellte, D-Pantothensäure-haltige Fermentationsbrühe gegebenenfalls zunächst durch bekannte Separationsmethoden wie zum Beispiel der Zentrifugation, der Filtration, dem Dekantieren oder einer Kombination hieraus ganz oder zum Teil von der Biomasse befreit. Es ist erfindungsgemäß jedoch auch möglich, die Biomasse gänzlich in der Fermentationsbrühe zu belassen. Anschließend wird die gegebenenfalls vorbehandelte Brühe mit bekannten Methoden zum Beispiel mittels eines Rotationsverdampfers oder Dünnschichtverdampfers oder Fallfilmverdampfers eingedickt oder getrocknet. Die gegebenenfalls aufkonzentrierte Brühe wird anschließend durch Methoden der Sprühtrocknung, Sprühgranulation oder Gefriertrocknung oder durch anderweitige Verfahren zu einem rieselfähigen, frei fließenden, feinteiligen Pulver oder Granulat verarbeitet.

Die erfindungsgemäßen neuen Tierfuttermittel-Additive enthalten 20 - 80 Gew.-%, vorzugsweise 30 - 75 Gew.-% D-Pantothensäure und/oder deren Salze, bezogen auf die Gesamtmenge. Sie enthalten zusätzlich im allgemeinen anorganische Bestandteile mit einer Menge von 2,5 - 25 Gew.-% und gegebenenfalls organische Nebenprodukte in einer Menge von > 0 bis 30 Gew.-%. Der Anteil an Biotrockenmasse beläuft sich auf 0 bis 35 Gew.-%. Der Wassergehalt liegt bevorzugt bei 5 5 Gew.-%. Der gewünschte Gehalt an D-Pantothensäure und/oder einem oder mehreren der genannten Salze wird gegebenenfalls durch Zusatz der entsprechenden Verbindungen zu den fermentativ hergestellten Produkten erzielt. Die gewünschten Verbindungen werden bevorzugt vor der Trocknung oder Sprühtrocknung, insbesondere nach der Aufkonzentrierung, dem Gemisch in Form von Lösungen oder Trockensubstanz zugesetzt und mit diesen vermischt. Das so erhaltene Produkt wird als Futtermitteladditiv eingesetzt.

Die Konzentration an D-Pantothensäure kann mit bekannten Verfahren (Velisek; Chromatographic Science 60, 515-560 (1992)) bestimmt werden.

### Beispiele

Die vorliegende Erfindung wird im folgenden anhand von Ausführungsbeispielen näher erläutert. Zu diesem Zweck wurden Versuche mit dem D-Pantothensäure produzierenden Stamm Escherichia coli 5069/pFV31 durchgeführt, der als FERM-BP 4395 gemäss Budapester Vertrag beim Fermentation Research Institute, Agency of Industrial Science and Technology in 1-1-3, Higashi, Tsukuba-shi, Ibaraki (Japan) hinterlegt ist.

### Beispiel 1

### Herstellung einer D-Pantothensäure haltigen Fermentationsbrühe

### 1. Herstellung des Inokulums

Eine Probe von Escherichia coli FV5069/pFV31 wurde auf LBG-Agar ausgestrichen, der mit 50 µg pro ml Ampicillin supplementiert worden war. Diese Agarplatten-Kultur wurde 17 Stunden bei 37°C inkubiert und dann im Kühlschrank bei +4°C aufbewahrt.Ausgewählte Einzelkolonien wurden anschließend in LBG-Bouillion weiter vermehrt. LBG-Bouillion hat folgende Zusammensetzung: 10 g/l Pepton, 5 g/l Hefeextrakt, 5 g/l NaCl und 1g/l Glucose. LBG-Agar enthält zusätzlich 12 g/l Agar. Vorgefertigte Zubereitungen können von der Firma Gibco/BRL (Paisley, Schottland, Grossbritanien) als LB Broth Base oder LB-Agar bezogen werden. Nach Zusatz von 1 g/l Glucose erhält man dann die angegebenen Medien. Kulturen von 10 ml, die in 100 ml Erlenmeierkolben enthalten waren, wurden für 16 Stunden bei 37°C und 180 rpm auf einem ESR Inkubator der Firma Kühner AG (Birsfelden,Schweiz) inkubiert. Im Anschluß wurde die Zellsuspension auf einer J-6B Zentrifuge der Firma Beckmann (Hannover,Deutschland) 15 Minuten bei 4000 rpm abzentrifugiert. Das Zellpellet wurde in 10 ml LBG-Medium, das mit 20% Glycerin supplementiert worden war, resuspendiert und in 10 Aliquots je 1 ml unter sterilen Bedingungen abgefüllt und bei -70°C eingefroren. Diese Kulturen wurden als "master"-Zellbank (mäster cell bank) verwendet.

Für die Herstellung einer Arbeitszellbank (working cell bank) wurde LBG-Medium, das mit 50 µg/ml Ampicillin supplementiert worden war, in 10 ml Portionen in 100 ml Erlenmeyerkolben verteilt und anschließend mit 100 µl der oben beschriebenen "master"-Zellbank beimpft. Die Inkubation erfolgte für 16 Stunden bei 37°C und 180 rpm auf einem ESR Inkubator der Firma Kühner AG (Birsfelden,Schweiz). Nach der Inkubation wurde die optische Dichte (OD) der Kultursuspension mit einem LP2W-Photometer der Firma Dr. Lange (Berlin, Deutschland) bei einer Messwellenlänge von 660 nm bestimmt. Sie betrug 3,5. Anschließend wurde die Zellsuspension in sterilen 30 ml Polyethylenröhrchen der Firma Greiner (Frickenhausen, Deutschland) unter sterilen Bedingungen abgefüllt und bei 2500 rpm für 15 Minuten mit einer Zentrifuge vom Typ J-6B der Firma Beckmann (Hannover, Deutschland) abzentrifugiert. Die abgetrennte Biomasse wurde in 10 ml LBG-Medium, das mit 20% Glycerin supplementiert worden war, resuspendiert. Im Anschluß wurde die Zellsuspension in 500 µl Portionen in 1 ml sterilen, der Firma Nalgene (New York, U.S.A.) unter sterilen Bedingungen abgefüllt und bei -70°C eingefroren. Die auf diese Weise hergestellten Konserven wurden als Arbeitszellbank (working cell bank) verwendet.

### 2. Herstellung einer D-Pantothensäure haltigen Fermentationsbrühe

Zur Herstellung einer Pantothensäure-haltigen Fermentationsbrühe wurde die Arbeitszellbank zunächst in einer Schüttelkolbenkultur vermehrt und diese zur Beimpfung eines Vorfermenters verwendet. Die Kultur des Vorfermenters wurde zur Beimpfung des Produktionsfermenters verwendet.

Für die Schüttelkolbenkultur wurde das Medium SKA verwendet. Medium SKA wurde folgendermassen zubereitet. In einem 1 l Becherglas wurden 7,0 g (NH₄)₂SO₄, 0,5 g KH₂PO₄, 1,0 g K₂HPO₄, 0,5 g MgSO₄*7 H₂O, 0,01 g MnSO₄*H₂O, 0,01 g ZnSO₄*7H₂O, 0,005 g Fe₂(SO₄)₃, und 20 g Maisquellwasser abgewogen, das zuvor mit 25%-iger Ammoniaklösung auf pH 6,8 eingestellt worden war, und anschliessend 875 ml destilliertes Wasser dazugegeben. Diese Maisquellwasser haltige Salzlösung wurde im Autoklav bei 121°C für 20 Minuten sterilisiert. Weiterhin wurde eine Lösung bestehend aus 125 g destilliertem Wasser, 28,7 g Glucose und 0,002 g Thiamin*HCl durch Filtration sterilisiert. 10 g CaCO₃ wurden in einem 100 ml Kolben abgewogen und im Autoklaven bei 123°C für 20 Minuten sterilisiert. Durch Vereinigung der beiden oben genannten Komponenten mit der Maisquellwasser haltigen Salzlösung wurde das Medium SKA erhalten.

Dieses Medium SKA wurde in 12,5 ml Portionen in 100 ml Erlenmeyerkolben verteilt und anschließend mit 0,5 ml einer Zellsuspension beimpft. Als Zellsuspension wurde eine mit steriler physiologischer Kochsalzlösung 1:100 verdünnte Konserve der Arbeitszellkultur verwendet. Die Inkubation erfolgte für 20 Stunden bei 32°C und 150 rpm auf einem RC-1-TK Inkubator der Firma Infors AG (Bottmingen, Schweiz). Die im Anschluß daran bestimmte optische Dichte bei einer Messwellenlänge von 660 nm (OD 660) betrug 12,5.

0,5 ml dieser Schüttelkolbenkultur wurden mit 4,5 ml physiologischer Kochsalzlösung verdünnt und davon 0,7 ml zum Beimpfen von 1300 ml Anzuchtsmedium verwendet, die in einem 21 Laborfermenter vom Typ Biostat® MD der Firma Braun Diessel Biotech GmbH (Melsungen, Deutschland) vorlagen.

Das Anzuchtmedium wurde folgendermassen zubereitet. Eine Lösung bestehend aus 9,81 g (NH₄)₂SO₄, 0,7 g KH₂PO₄, 1,402 g K₂HPO₄, 0,70 g MgSO₄*7 H₂O, 0,014 g MnSO₄*H₂O, 0,014g Fe₂(SO₄)₃, und 28,04 g Maisquellwasser in 1300 ml Leitungswasser wurde auf pH 6,5 mit 25%iger Ammoniaklösung eingestellt und im Autoklaven bei 121°C für 20 Minuten sterilisiert. Zu dieser Maisquellwasser haltigen Salzlösung wurde eine separat sterilfiltrierte Lösung, die in 100g destilliertem Wasser 40,62 g Glucose und 0,0042 g Thiamin*HCl enthielt, unter sterilen Bedingungen hinzugegeben.

Die Fermentation erfolgte für 16 Stunden bei 37°C und einer Belüftung von 1 vvm. Der Gelöstsauerstoff wurde bei 20% und der pH bei 6,5 gehalten. Als pH-Korrekturmittel wurde 25%ige Ammoniaklösung eingesetzt. Die optische Dichte betrug 13,1. 90 ml dieser Kultur wurde zum Beimpfen von 1144 ml Wachstumssmedium für die Hauptfermentation in einem 2 l Laborfermenter vom Typ Biostat® MD verwendet.

Das Wachstummedium wurde folgendermassen zubereitet. Eine Lösung bestehend aus 4,14 g (NH₄)₂SO₄, 0,744 g KH₂PO₄, 1,0 g K₂HPO₄, 0,83 g MgSO₄*7 H₂O, 0,0124 g MnSO₄*H₂O, 18,87 g β-Alanin, 0,74 g Struktol J647 und 49,72 g Maisquellwasser in 1144 ml Leitungswasser, wurde auf pH 6,5 mit 25%iger Ammoniaklösung eingestellt und im Autoklaven bei 121°C für 20 Minuten sterilisiert. Zu dieser Maisquellwasser haltigen Salzlösung wurde eine separat sterilfiltrierte Lösung, die in 100 ml destilliertem Wasser 35,92 g Glucose, und 0,002 g Thiamin*HCl enthielt, unter sterilen Bedingungen hinzugegeben.

Die Fermentation erfolgte für 40 Stunden bei 37°C. In der Wachstumsphase betrug der pH 6,5 und die Belüftung 1 vvm. In der Produktionsphase betrug der pH 6,0 und die Belüftung 1,5 vvm. Der Gelöstsauerstoff wurde in beiden Phasen unter 2% gehalten.. Als pH Korrekturmittel wurde die 25%ige Ammoniaklösung verwendet. Während der Fermentation wurden das Produktionsmedium 1, das Produktionsmedium 2 stufenweise zugefüttert. Maisquellwasser wurde während der Kultivierung einmalig zugegeben. Das Produktionsmedium enthält in 584 ml Leitungswasser 465,29 g Glucose und 0,0261 g Thiamin*HCl, die sterilfiltriert wurden. Das Produktionsmedium 2 enthält 37,5 g β- Alanin in 140 ml Leitungswasser, welches bei 121°C 20 Minuten im Autoklaven sterilisiert wurde. Nach einer Fermentationszeit von 7,5 Stunden bis zum Ende der Kultivierung wurde das Produktionsmedium 1 stufenweise zugefüttert. Nach 10,5 Stunden Kultivierung wurden zusätzliche 49,5 g Maisquellwasser, welches in 100 ml Leitungswasser gelöst waren und für 20 Minuten bei 121°C im Autoklav sterilisiert wurden, unter sterilen Bedingungen zugegeben. Nach einer Fermentationszeit von 12,5 Stunden bis zum Ende der Kultivierung wurde das Produktionsmedium 2 mit einer Dosierrate von 3,5 g/h zugefüttert. Nach 41 Stunden der Kultivierung wurde in der Fermentationsbrühe eine Pantothensäurekonzentration 6,1 Gew.-% gefunden.

Das Gehalt an D-Pantothensäure wurde mit Hilfe einer HPLC-(Hochleistung Flüssig Chromotographie) Anlage vom Typ M321 der Firma Knauer (Berlin, Deutschland) mittels RI-(Refraction Index) Detektion unter Verwendung einer Hypersil APS2 Aminophase von 5 um Korngrösse bestimmt.

### Beispiel 2

Bei einem Fermentationsversuch, der unter gleichen Bedingungen, wie in Beispiel 1 beschrieben, durchgeführt wurde, konnte nach einer Kultivierungszeit von 43 Stunden eine Pantothensäurekonzentration von 5,4 Gew.-% in der Fermentationsbrühe nachgewiesen werden. Die Konzentration an L-Valin betrug 8 g/l.

### Beispiel 3

### Herstellung von D-Calcium-Pantothenat

Aus einer pantothensäurehaltigen Fermentationsbrühe, die nach den Verfahren gemäß den Beispielen 1 und 2 hergestellt wurde, und die etwa 6,1 Gew.-% D-Pantothensäure enthielt, wurde zunächst die Biomasse abgetrennt. Hierzu wurde 1 l der oben genannten Fermentationsbrühe mit einer Laborzentrifuge vom Typ Biofuge-Stratos der Firma Heraeus (Düsseldorf, Deutschland) für 20 Minuten bei 4.000 rpm zentrifugiert und der Zentrifugationsüberstand anschließend durch Cross-Flow Ultrafiltration mit einer MRC Polymermembran von 30kD in einer UF-Anlage der Firma ICT GmbH (Bad Homburg, Deutschland) weiter gereinigt.

Anschließend wurde unter Rühren absatzweise 10,1 g festes Ca(OH)₂ (96%; Firma MERCK, Darmstadt, Deutschland) zugegeben. Der pH-Wert betrug danach ca. 10,3. Die so behandelte Brühe wurde dann unter Vakuum bei 60°C in einem Rotationsverdampfer vom Typ Rotavapor RE-120 der Firma Büchi-Labortechnik GmbH (Konstanz, Deutschland) auf einen Flüssigkeitsanteil von etwa 50% Trockengehalt eingeengt. Die so eingeengte Brühe wurde anschließend zur Darstellung des Calciumsalzes der D-Pantothensäure sprühgetrocknet. Hierzu wurde ein Laborsprühtrockner vom Typ Büchi-190 der Firma Büchi-Labortechnik GmbH (Konstanz, Deutschland) bei einer Eingangstemperatur von 107°C, einer Ausgangstemperatur von 85°C, einer Druckdifferenz von -40 mbar und einer Luftstromgeschwindigkeit von 600 NL/h eingesetzt.

Das so hergestellte Calcium D-Pantothenat haltige Produkt besaß einen Gehalt von 68,5 Gew.-% D-Pantothensäure, war rieselfähig und hatte eine Schüttdichte von 460 mg/ml. Nach einer Lagerzeit von fünf Monaten betrug der Gehalt an D-Pantothensäure 67,6 Gew.-%.

### Beispiel 4

### Herstellung von D-Natrium-Pantothenat

Aus einer pantothensäurehaltigen Fermentationsbrühe, die nach den Verfahren gemäß den Beispielen 1 und 2 hergestellt wurde, und die etwa 6,1 Gew.-% D-Pantothensäure enthielt, wurde zunächst die Biomasse abgetrennt. Hierzu wurde 1 l der oben genannten Fermentationsbrühe so, wie in Beispiel 3 beschrieben, zentrifugiert und ultrafiltriert.

Anschließend wurde unter Rühren absatzweise 10,6 g NaOH (99%; Firma MERCK) zugegeben. Der pH-Wert betrug danach etwa 10. Die so behandelte Brühe wurde dann unter Vakuum bei 50- 60°C in einem Rotationsverdampfer vom Typ Rotavapor RE-120 der Firma Büchi-Labortechnik GmbH auf einen Flüssigkeitsanteil von etwa 50% Trockengehalt eingeengt. Die so eingeengte Brühe wurde anschließend zur Darstellung des Natriumsalzes der D-Pantothensäure in einem Gefriertrockner vom Typ LYOVAC GT 2 der Firma Leybold (Köln, Deutschland) lyophilisiert.

Das so hergestellte Natrium D-Pantothenat haltige Produkt besaß einen Gehalt von 63,8 Gew.-% D-Pantothensäure und war rieselfähig. Nach einer Lagerzeit von fünf Monaten betrug der Gehalt an D-Pantothensäure 63,0 Gew.-%.

### Beispiel 5

### Herstellung von D-Magnesium-Pantothenat

Aus einer pantothensäurehaltigen Fermentationsbrühe, die nach dem Verfahren von Beispiel 1 und 2 hergestellt wurde und die etwa 6,1 Gew.-% D-Pantothensäure enthielt, wurde zunächst die Biomasse abgetrennt. Hierzu wurde 1 l der oben genannten Fermentationsbrühe so wie in Beispiel 2 beschrieben zentrifugiert und ultrafiltriert.

Anschließend wurde unter Rühren absatzweise 5,4 g festes MgO (97%; Firma MERCK) zugegeben. Der pH-Wert betrug danach etwa 9 bis 10. Die so behandelte Brühe wurde dann unter Vakuum bei 50- 60°C in einem Rotationsverdampfer vom Typ Rotavapor RE-120 der Firma Büchi-Labortechnik GmbH auf einen Flüssigkeitsanteil von etwa 50% Trockengehalt eingeengt. Die so eingeengte Brühe wurde anschließend zur Darstellung des Magnesiumsalzes der D-Pantothensäure in einem Gefriertrockner vom Typ LYOVAC GT 2 der Firma Leybold lyophilisiert.

Das so hergestellte Magnesium D-Pantothenat-haltige Produkt besaß einen Gehalt von 64,7 Gew.-% D-Pantothensäure und war rieselfähig. Nach einer Lagerzeit von fünf Monaten betrug der Gehalt an D-Pantothensäure 64,4 Gew.-%.

### Beispiel 6

### Herstellung von D-Kalium-Pantothenat

Aus einer pantothensäurehaltigen Fermentationsbrühe, die nach den Verfahren gemäß den Beispielen 1 und 2 hergestellt wurde, und die etwa 6,1 Gew.-% D-Pantothensäure enthielt, wurde zunächst die Biomasse abgetrennt. Hierzu wurde 1 l der oben genannten Fermentationsbrühe so, wie in Beispiel 2 beschrieben, zentrifugiert und ultrafiltriert.

Anschließend wurde unter Rühren absatzweise 17,4 g KOH (85%; Firma MERCK) zugegeben. Der pH-Wert betrug danach etwa 10 bis 11. Die so behandelte Brühe wurde dann unter Vakuum bei 60°C in einem Rotationsverdampfer vom Typ Rotavapor RE-120 der Firma Büchi-Labortechnik GmbH auf einen Flüssigkeitsanteil von etwa 50% Trockengehalt eingeengt. Die so eingeengte Brühe wurde anschließend zur Darstellung des Kaliumsalzes der D-Pantothensäure in einem Gefriertrockner vom Typ LYOVAC GT 2 der Firma Leybold lyophilisiert.

Das so hergestellte Kalium D-Pantothenat haltige Produkt besaß einen Gehalt von 63,5 Gew.-% D-Pantothensäure und war rieselfähig. Nach einer Lagerzeit von fünf Monaten betrug der Gehalt an D-Pantothensäure 62,9 Gew.-%.

### Beispiel 7

### Herstellung von D-Ammonium-Pantothenat

Aus einer pantothensäurehaltigen Fermentationsbrühe, die nach den Verfahren gemäß den Beispielen 1 und 2 hergestellt wurde und die etwa 6,1 Gew.-% D-Pantothensäure enthielt, wurde zunächst die Biomasse abgetrennt. Hierzu wurde 1 l der oben genannten Fermentationsbrühe so, wie in Beispiel 2 beschrieben, zentrifugiert und ultrafiltriert.

Die so behandelte Brühe wurde dann unter Vakuum bei 60°C in einem Rotationsverdampfer vom Typ Rotavapor RE-120 der Firma Büchi-Labortechnik GmbH auf einen Flüssigkeitsanteil von etwa 50% Trockengehalt eingeengt. Die so eingeengte Brühe wurde anschließend zur Darstellung des Ammoniumsalzes der D-Pantothensäure in einem Gefriertrockner vom Typ LYOVAC GT 2 der Firma Leybold lyophilisiert.

Das so hergestellte Ammonium D-Pantothenat haltige Produkt besaß einen Gehalt von 66,8 Gew.-% D-Pantothensäure und war rieselfähig.

### Beispiel 8

### Herstellung von D-Calcium-Pantothenat aus einer biomassehaltigen Fermentationsbrühe

Eine pantothensäurehaltigen Fermentationsbrühe, die nach den Verfahren gemäß den Beispielen 1 und 2 hergestellt wurde, und die etwa 6,1 Gew.-% D-Pantothensäure enthielt, wurde zunächst unter Vakuum bei 60°C in einem Rotationsverdampfer vom Typ Rotavapor RE-120 der Firma Büchi-Labortechnik GmbH (Konstanz, Deutschland) ein Volumen von 1,0 l auf einen Flüssigkeitsanteil von etwa 30% Trockengehalt eingeengt. Anschließend wurde unter Rühren absatzweise 10,1 g festes Ca(OH)₂ (96%; Firma MERCK, Darmstadt, Deutschland) zugegeben. Der pH-Wert betrug danach ca. 10. Die so behandelte und eingeengte biomassehaltige Brühe wurde anschließend zur Darstellung des Calciumsalzes der D-Pantothensäure sprühgetrocknet. Hierzu wurde ein Laborsprühtrockner vom Typ Büchi-190 der Firma Büchi-Labortechnik GmbH (Konstanz, Deutschland) bei einer Eingangstemperatur von 107°C, einer Ausgangstemperatur von 85°C, einer Druckdifferenz von -40 mbar und einer Luftstromgeschwindigkeit von 600 NL/h eingesetzt.

Das so hergestellte Calcium D-Pantothenat haltige Produkt besaß einen Gehalt von 49,8 Gew.-% D-Pantothensäure, war rieselfähig und hatte eine Schüttdichte von 480 mg/ml. Der Biomasse-Gehalt betrug ca. 30 Gew.-%.

### Beispiel 9

### Herstellung eines D-Calcium-Pantothenat und Biomasse von Corynebacterium glutamicum enthaltenden Produktes

### 1. Herstellung eines Pantothensäure produzierenden Stammes von Corynebacterium glutamicum

In der Patentschrift US-A-5,188,948 ist der L-Valin produzierende Stamm Brevibacterium lactofermentum FERM BP-1763 beschrieben. Aus der DE 19855313.7 ist das Plasmid pND-DBC2 (Abbildung 1) bekannt, welches die Gene panB, panC und panD von Corynebacterium glutamicum trägt. Das Plasmid ist in Form des Stammes ATCC13032/pND-DBC2 als DSM 12437 bei der Deutschen Sammlung für Mikroorganismen und Zellkulturen (Braunschweig, Deutschland) hinterlegt. Durch Transformation des Stammes FERM BP-1763 mit dem Plasmid pND-DBC2 entstand der Pantothensäure produzierende Stamm FERM BP-1763/pND-DBC2.

### 2. Herstellung einer Pantothensäure haltigen Fermentationsbrühe

Eine Probe von Brevibacterium lactofermentum FERM BP-1763/pND-DBC2 wurde auf HHK-Agar ausgestrichen.

HHK-Agar besteht aus Hirn-Herz-Agar, der von der Firma Merck KgaA (Darmstadt, Deutschland) bezogen und mit Kanamycin supplementiert wurde. Die Zusammensetzung des HHK-Agars ist in Tabelle 8a angegeben.

Diese Agarplatten-Kultur wurde 17 Stunden bei 37°C inkubiert und dann im Kühlschrank bei +4°C aufbewahrt. Ausgewählte Einzelkolonien wurden anschließend auf dem gleichen Medium weiter vermehrt. Mit einer Impföse wurde Zellmaterial eines Klons vom HHK-Agar abgenommen und in 100 mL HHK-Bouillion übertragen, die in einem Schüttelkolben von 1000 mL Gesamtvolumen enthalten waren.

HHK-Bouillion besteht aus Hirn-Herz-Medium, das von der Firma Merck KgaA (Darmstadt, Deutschland) bezogen und mit Glucose und Kanamycin supplementiert wurde. Die Zusammensetzung der HHK-Bouillion ist in Tabelle 8b angegeben.

**Tabelle 8a**

| HHK-Agar | |
|---|---|
| Substanz | Menge pro Liter |
| Hirn-Herz-Agar | 52,0 g |
| Kanamycin | 25 mg |

**Tabelle 8b**

| HHK-Boullion | |
|---|---|
| Substanz | Menge pro Liter |
| Hirn-Herz-Medium | 37,0 g |
| Kanamycin | 25 mg |
| Glucose | 20,0 g |

Der Ansatz wurde bei 30°C und 150 rpm für 22 Stunden inkubiert. Nach Ende der Kultivierung wurde im Photometer bei einer Wellenlänge von 660 nm (OD 660) eine optische Dichte von 6,1 gemessen. Diese Kultur des Stammes FERM BP-1763/pND-DBC2 wurde zur Beimpfung des Produktionsfermenters verwendet.

Zur Fermentation wurde das in Tabelle 8c angegebene Medium SK-71 verwendet. Alle Komponenten des SK-71-Mediums wurden direkt entsprechend der Arbeitskonzentrationen im Fermenter vorgelegt und in situ sterilisiert.

**Tabelle 8c**

| Medium SK-71 | |
|---|---|
| Verbindung | Menge pro Liter |
| Glucose Hydrat | 110,0000g |
| Cornsteep Liquor (CSL) | 5,0000g |
| β-Alanin | 5,0000g |
| Nicotinsäure | 0,0050g |
| l-Isoleucin | 0,1500g |
| Homoserin | 0,1500g |
| Ammoniumsulfat | 25,0000g |
| K-dihydrogenphosphat | 0,1000g |
| Mg-Sulfat 7H₂O | 1,0000g |
| Fe-Sulfat 7H₂O | 0,0100g |
| Mn-Sulfat H₂O | 0,0050g |
| CaCl₂ * 2H₂O | 0,0100g |
| Thiamin HCl | 0,0002g |
| D(+)Biotin | 0,0003g |
| Struktol | 0,60g |

Als Fermenter wurden 10 l Rührreaktoren der Firma B.Braun (BBI, Deutschland, Melsungen, Modell Biostat E/ED) verwendet.

Zur Inokulierung von 1950 g des Fermentationsmediums SK-71 wurden 100 mL der oben beschriebenen Schüttelkolbenvorkultur in HHK-Bouillion eingesetzt.

Der Ansatz wurde über die gesamte Fermentationsdauer bei einer Temperatur von 30°C, einer volumenspezifischen Belüftung von 0,75 vvm, einer vom Sauerstoffverbrauch abhängigen Rührung von 800 - 1700 rpm und einem pH von 7,0 und einem Sauerstoffpartialdruck von 20% der Luftsättigung kultiviert. Die Kultur wurde insgesamt für 49 Stunden unter den obengenannten Bedingungen bis zum Erreichen einer OD660 von 26,2 kultiviert. Als Korrekturmittel zur pH-Wertregulierung wurde eine wässrige Ammoniak-Lösung (25 % w/v) verwendet.

Anschließend wurden die optische Dichte (OD) mit einem Digitalphotometer vom Typ LP1W der Firma Dr. Bruno Lange GmbH (Berlin, Deutschland) bei einer Meßwellenlänge von 660 nm und die Konzentration an gebildeter D- Pantothensäure mittels HPLC(Hypersil APS 2 5 µm, 250x5 mm, RI-Detektion) bestimmt.

In der Fermentationsendprobe wurde nach 49 Stunden eine D-Pantothensäure Konzentration von ca. 0,2 g/l gemessen.

### 3. Herstellung eines Pantothensäure haltigen Produktes

Eine D-Pantothensäure haltige Fermentationsbrühe mit einem Gehalt von etwa 0,02 Gew.-% D-Pantothensäure wurde nach dem Verfahren von Beispiel 9.2 hergestellt. Ein Volumen von 1,4 l dieser Fermentationsbrühe wurde zunächst unter Vakuum bei 60°C in einem Rotationsverdampfer vom Typ Rotavapor RE-120 der Firma Büchi-Labortechnik GmbH (Konstanz, Deutschland) auf eine Brühe von etwa 15% Trockengehalt eingeengt. Anschließend wurde unter Rühren absatzweise 27,1 g festes Ca(OH)₂ (96%; Firma MERCK, Darmstadt, Deutschland) zugegeben. Der pH-Wert betrug danach ca. 10,0. Die so behandelte und eingeengte biomassehaltige Brühe wurde anschließend mit 37,7 g Calcium D-Pantothenat (>98 %, Euro OTC Pharma GmbH, Kamen, Deutschland) versetzt. Für die anschließende Sprühtrocknung wurde ein Laborsprühtrockner vom Typ Büchi-190 der Firma Büchi-Labortechnik GmbH (Konstanz, Deutschland) bei einer Eingangstemperatur von 107°C, einer Ausgangstemperatur von 85°C, einer Druckdifferenz von -40 mbar und einer Luftstromgeschwindigkeit von 600 NL/h eingesetzt.

Das so hergestellte Calcium D-Pantothenat haltige Produkt besaß einen Gehalt von ca. 35 % D-Pantothensäure, war rieselfähig und hatte eine Schüttdichte von 600 mg/ml. Der Anteil an C. glutamicum - Biomasse betrug ca. 3,5 Gew.-%.

### Beispiel 10

### Herstellung eines D-Calcium-Pantothenat und Biomasse von Saccharomyces cerevisiae enthaltenden Produktes

### 1. Herstellung eines Pantothensäure produzierenden Stammes von Saccharomyces cerevisiae

### Amplifikation des Leserasters YHR063c:

Ausgehend von der Nukleotidsequenz des Saccharomyces cerevisiae Leserasters YHR063c (Accession Nr. U00061 des National Center for Biotechnology, Bethesda, MD, USA) wurden die nachstehenden PCR-Primer synthetisiert (MWG-Biotech, Ebersberg, Deutschland). Anfang bzw. Ende des Leserasters sind durch einen Punkt (.) gekennzeichnet:
- oJD539 (5' EcoRI-NotI ST ART): 
5'- GCG CGA ATT CAG ATC CGC GGC CGC AAA GAG GAG AAA TTA
ACT.ATG ACT GCA CCA CAC AGA AG -3'
- oJD540 (3' SpeI-PstI STOP):

5'- CGC GAC TAG TCT GCA G.TC AGT CCT TTC TCC AGT CAC-3'

Als Template diente genomische DNA des S. cerevisiae Stammes JD242, die nach der Methode von C. Guthrie und G.R. Fink (Guide to yeast genetics and molecular biology, Methods in Enzymology, Vol. 194, Academic Press, San Diego, CA, 1991) isoliert wurde. Dieser Stamm ist eine haploide Segregante des diploiden Stammes SC288C (Winston et al., Yeast 11, 53ff. (1995)), dessen Genom sequenziert wurde (Goffeau et al., Science 274, pp. 546, (1996)). Die Tetradenanalyse erfolgte nach der Methode von C. Guthrie und G.R. Fink (Guide to yeast genetics and molecular biology, Methods in Enzymology, Vol. 194, Academic Press, San Diego, CA, 1991). Der Stamm JD242 ist auxotroph für Leucin (leu2Δ1 Allel) und Uracil (ura3-52 Allel). Ein etwa 1,2 kB großes DNA-Fragment konnte unter Verwendung des "High Fidelity Expand Polymerase" Kits der Firma Roche (Mannheim) durch 28 PCR-Zyklen unter den vom Hersteller angegebenen Bedingungen amplifiziert werden. Die Größe wurde durch elektrophoretische Auftrennung in einem 0,8%igen Agarosegel bestimmt.

### Konstruktion von pJD-YHR063c:

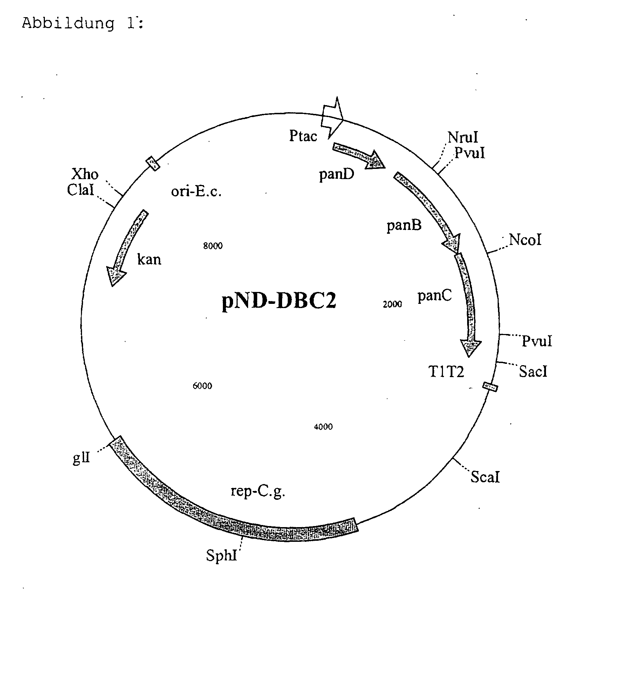

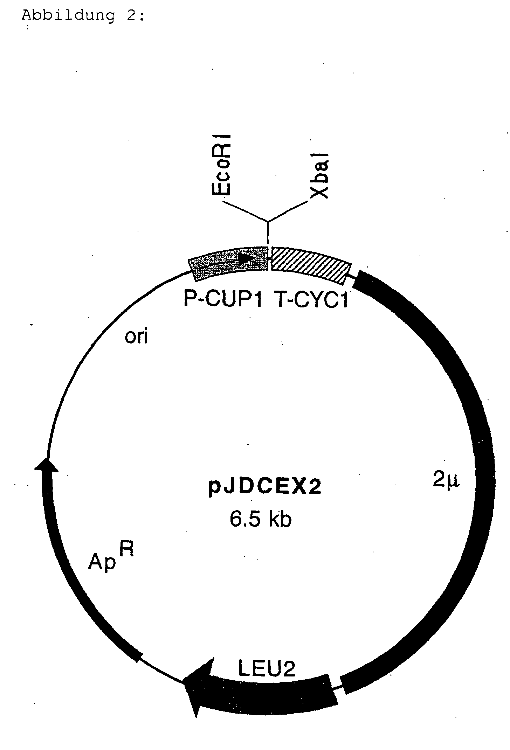

Zur Expression des YHR063c Leserasters in S. cerevisiae wurde das PCR-Amplifikat in den E. coli - S. cerevisiae Pendelvektor pJDCEX2 eingebaut (Abbildung 2 und Dohmen et al., 1995, Journal of Biological Chemistry 270, 18099-18109).

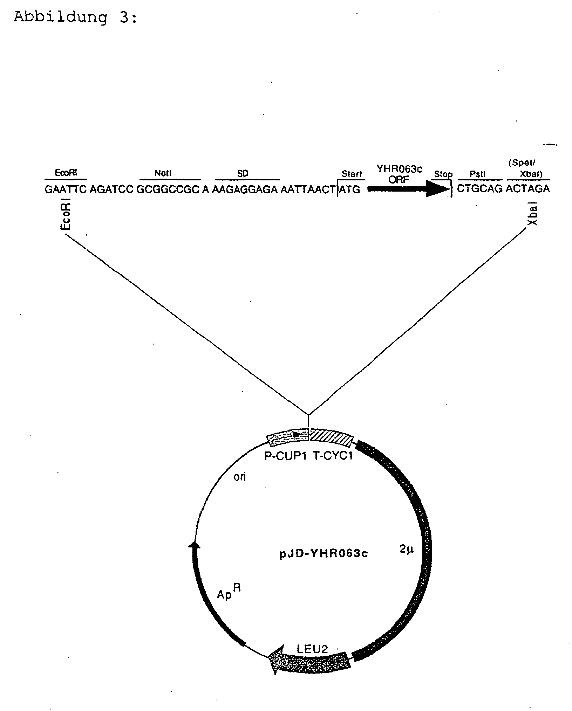

Das PCR-Produkt wurde zunächst mit EcoRI und SpeI (AGS, Heidelberg, Deutschland) restringiert. Anschließend wurde es mit pJDCEX2-DNA, welche mit EcoRI und XbaI (AGS, Heidelberg, Deutschland) behandelt worden war, gemischt und mit T4-DNA Ligase (Roche, Mannheim, Deutschland) ligiert. Der Ligationsansatz wurde in den E. coli Stamm XL1-Blue (Bullock et al., 1987, Biotechniques 5, 376) transformiert. Tranformanten wurde durch Selektion auf LB-Agar, welcher 150 µg/ml Ampicillin (Sigma, Deisenhofen, Deutschland) enthielt, erhalten. Die Plasmid-DNA aus den Ampicillin resistenten Klonen wurde durch alkalische Lyse präpariert (Sambrook et al.: Molecular Cloning, A Laboratory Manual, Cold Spring Harbor Laboratory Press, 1989). Die isolierte Plasmid-DNA wurde dann durch Restriktion mit NotI und PstI und anschließender Auftrennung im 0,8%igen Agarosegel untersucht. Das Plasmid mit der gewünschten Struktur erhielt den Namen pJD-YHR063c (Abbildung 3).

### Herstellung von Stamm JD242/pJD-YHR063c:

Der S. cerevisiae Stamm JD242 wurde mit dem Plasmid pJD-YHR063c nach der Methode von Dohmen et al. transformiert (Dohmen et al., Yeast 7, 691 (1991)). Die Selektion auf Transformanten erfolgte auf leucinfreiem Minimalmedium SD mit 1,8% Agar(siehe Tabelle 9a und 9b).

**Tabelle 9a**

| Minimalmedium SD | |
|---|---|
| Verbindung | Menge pro Liter |
| (NH₄)₂ SO₂ | 5 g |
| KH₂PO₄ | 1 g |
| MgSO₄ * 7 H₂O | 0,5 g |
| NaCl | 0,1 g |
| CaCl₂ | 0,1 g |
| H₃BO₃ | 500 µg |
| CuSO₄ | 40 µg |
| KI | 100 µg |
| FeCl₃ * 6 H₂O | 200 µg |
| MnSO₄ * H₂O | 400 µg |
| Na₂MoO₄ * 2 H₂O | 400 µg |
| ZnSO₄ * 7 H₂O | 200 µg |
| Biotin | 2 µg |
| Folsäure | 2 µg |
| Inositol | 2 mg |
| Niacin | 400 µg |
| p-Aminobenzoesäure | 200 µg |
| Pyridoxin Hydrochlorid | 400 µg |
| Riboflavin | 200 µg |
| Thiamin Hydrochlorid | 400 µg |

**Tabelle 9b**

| Minimalmedium SD | |
|---|---|
| Zusätze | Menge pro Liter |
| Glucose | 20 g |
| Uracil | 40 mg |
| CuSO₄ | 24 mg |
| -Leu DO Supplement | 650 mg |
| Ketopantoat | 100 mg |
| β-Alanin | 100 mg |

### 2. Herstellung einer Pantothensäure haltigen Fermentationsbrühe

Zur Herstellung einer D-Pantothenat haltigen Fermentationsbrühe wurde zunächst eine Einzelkolonie von S. cerevisiae Stamm JD242/pJD-YHR063c auf einer Agarplatte mit Minimalmedium SD ausgestrichen und 3 Tage bei 30°C inkubiert. Zur Herstellung dieser ersten Vorkultur im Schüttelkolben wurden anschließend die Zellen mit 5 mL des Minimalmediums SD abgeschwemmt. Mit 2,5 mL dieser Zellsuspension wurde dann jeweils ein Schüttelkolben (500 mL Gesamtvolumen) mit 50 mL Minimalmedium SD (Tabelle 9a und 9b) angeimpft und bei 30°C und 130 rpm auf einem RC-1-TK Inkubator der Firma Infors AG (Bottmingen, Schweiz) für 6 Stunden kultiviert, bis eine optische Dichte bei einer Wellenlänge von 660 nm (OD660) von 1,9 gemessen werden konnte. Die zweite Vorkultur wurde in einem 1.000 mL Schikanekolben in 150 mL Minimalmedium SD (Tabelle 9a und 9b) angesetzt und mit jeweils 50 mL der oben beschriebenen Vorkultur 1 beimpft. Die Inkubation erfolgte bei 30°C und 80 rpm für 20 Stunden, bis eine optische Dichte bei einer Wellenlänge von 660 nm (OD 660) von ca. 3,8 erreicht wurde. Die Hauptfermentation zur Produktion der Pantothensäure wurde im Rundkolben mit 6000 mL Gesamtvolumen in 1500 mL Minimalmedium SD (Tabelle 9a und 9b) durchgeführt. Hierzu wurde der Rundkolben mit jeweils 90 mL der Vorkultur 2 beimpft und anschließend bei 30°C und 60 rpm für 30 Stunden inkubiert.

Die optische Dichte (OD) wurde mit einem Digitalphotometer vom Typ LP1W der Firma Dr. Bruno Lange GmbH (Berlin; Deutschland) bei einer Meßwellenlänge von 660 nm gemessen. Die Bestimmung der Konzentration an gebildeter D-Pantothensäure erfolgte mit Hilfe des Stammes Lactobacillus plantarum ATCC® 8014 nach Angaben des Handbuchs "DIFCO MANUAL" der Firma DIFCO (Michigan, USA;, 10^{th} Edition, 1100-1102 (1984).

Die optische Dichte (OD660) betrug ca. 4 und der Gehalt an D-Pantothensäure etwa 1 mg/l.

### 3. Herstellung eines Pantothensäure haltigen Produktes

Eine pantothensäurehaltigen Fermentationsbrühe mit einem Gehalt von etwa 1 mg/l D-Pantothensäure wurde nach dem Verfahren von Beispiel 10.2 hergestellt. Ein Volumen von 6,0 1 wurde zunächst unter Vakuum bei 60°C in einem Rotationsverdampfer vom Typ Rotavapor RE-151 der Firma Büchi-Labortechnik GmbH (Konstanz, Deutschland) zu einer Brühe von etwa 16% Trockengehalt eingeengt. Anschließend wurde unter Rühren absatzweise 15,9 g festes Ca(OH)₂ (96%; Firma MERCK, Darmstadt, Deutschland) zugegeben. Der pH-Wert betrug danach ca. 9,2. Die so behandelte und eingeengte biomassehaltige Brühe wurde anschließend mit 28,4 g Calcium D-Pantothenat(>98 %, Euro OTC Pharma GmbH, Kamen, Deutschland) versetzt. Die Brühe wurde anschließend in einem Gefriertrockner vom Typ LYOVAC GT 2 der Firma Leybold (Köln, Deutschland) lyophilisiert.

Das so hergestellte Calcium D-Pantothenat haltige Produkt besaß einen Gehalt von 26,5 Gew.-% D-Pantothensäure, war rieselfähig und hatte eine Schüttdichte von 450 mg/ml. Der Anteil an S. cerevisiae- Biomasse betrug ca. 6,8 Gew.-%.

### Abbildungen:

Folgende Abbildungen sind beigefügt:

| | |
|---|---|
| Abbildung 1 | Karte des Plasmids pND-DBC2 |
| Abbildung 2 | Karte des Plasmids pJDCEX2 |
| Abbildung 3 | Karte des Plasmids pJD-YHR063c |

Die verwendeten Abkürzungen haben folgende Bedeutung:

| Zu Abbildung 1: | |
|---|---|
| rrnBT1T2 | Transkriptions-Terminator des rrnB-Gens |
| Ptac | tac Promotor |
| panB | Kodierbereich des panB Gens |
| panC | Kodierbereich des panC Gens |
| panD | Kodierbereich des panD Gens |
| rep-C.g. | DNA-Region für Replikation in C. glutamicum |
| oriV-E.c. | Ursprung für vegetativen Transfer in E. coli |
| kan | Resistenzgen für Kanamycin |
| BglII | Schnittstelle des Restriktionsenzyms BglII |
| ClaI | Schnittstelle des Restriktionsenzyms ClaI |
| NcoI | Schnittstelle des Restriktionsenzyms NcoI |
| NruI | Schnittstelle des Restriktionsenzyms NruI |
| PvuI | Schnittstelle des Restriktionsenzyms PvuI |
| SacI | Schnittstelle des Restriktionsenzyms SacI |
| SalI | Schnittstelle des Restriktionsenzyms SalI |
| ScaI | Schnittstelle des Restriktionsenzyms ScaI |
| SphI | Schnittstelle des Restriktionsenzyms SphI |
| XhoI | Schnittstelle des Restriktionsenzyms XhoI |

| Zu Abbildung 2 und 3: | |
|---|---|
| LEU2 | Beta-Isopropylmalat Dehydrogenase-Gen von Saccharomyces cerevisiae |
| 2µ | Sequenzen des endogenen 2µ Plasmides von Saccharomyces cerevisiae |
| Ap^{R} | Beta-Lactamase-Gen |
| P-CUP1 | Promoter des Saccharomyces cerevisiae CUP1-Gens (Metallothionein) |
| T-CYC1 | Terminator des CYC1-Gens (Cytochrom C) von Saccharomyces cerevisiae |
| SD | Shine Dalgarno Sequenz |
| EcoRI | Schnittstelle des Restriktionsenzyms EcoRI |
| NotI | Schnittstelle des Restriktionsenzyms NotI |
| SpeI | Schnittstelle des Restriktionsenzyms SpeI |
| XbaI | Schnittstelle des Restriktionsenzyms XbaI |

## Patentansprüche

1. D-Pantothensäure und deren Salze oder deren Salze enthaltende Tierfuttermittel-Additive auf Fermentationsbrühe-Basis,
**dadurch gekennzeichnet,**
**daß** sie
a) D-Pantothensäure und/oder eines ihrer Salze in einer Menge von 20 bis 80 Gew.% (Trockenmasse),
b) die während der Fermentation der D-Pantothensäure produzierenden Mikroorganismen gebildete Biomasse in einer Menge von 0 bis 100 %,
c) zumindest den überwiegenden Teil der weiteren Inhaltsstoffe der Fermentationsbrühe enthalten und
d) in fester, feinteiliger oder granulierter, fließfähiger Form vorliegen.

2. Tierfuttermittel-Additive gemäß Anspruch 1,
**dadurch gekennzeichnet,**
**daß** sie eines oder mehrere der Salze, ausgewählt aus der Gruppe der Natrium-, Kalium-, Ammonium-, Magnesiumoder Calciumsalze der D-Pantothensäure enthalten.

3. Tierfuttermittel-Additive gemäß Anspruch 1,
**dadurch gekennzeichnet,**
**daß** sie in sprühgetrockneter oder sprühgranulierter Form vorliegen.

4. Tierfuttermittel-Additive gemäß einem oder mehreren der vorhergehenden Ansprüche,
**dadurch gekennzeichnet,**
**daß** sie zusätzlich eine oder mehrere der L-Aminosäuren enthalten, ausgewählt aus der Gruppe L-Methionin, L-Lysin, L-Valin, L-Alanin, L-Threonin oder L-Tryptophan.

5. Verfahren zur Herstellung von D-Pantothensäure und/oder eines ihrer Salze in einer Menge von 20 bis 80 Gew.% (Trockenmasse) enthaltenden Futtermittel-Additiven aus Fermentationsbrühen,
**dadurch gekennzeichnet,**
**daß** man
a) aus D-Pantothensäure und/oder deren Salze enthaltenden Fermentationsbrühe gegebenenfalls vollständig oder teilweise die Biomasse und/oder einen Teil der weiteren Inhaltsstoffe abtrennt,
b) die so erhaltene Lösung bzw. Brühe mit dem Hydroxid oder Oxid eines Erdalkali- oder Alkalimetalls versetzt und
c) das so erhaltene Gemisch trocknet oder sprühtrocknet, sprühgranuliert oder granuliert.

6. Verfahren gemäß Anspruch 5,
**dadurch gekennzeichnet,**
**daß** man ein Oxid oder Hydroxid, ausgewählt aus der Gruppe der Natrium-, Kalium-, Ammonium-, Magnesiumoder Calciumverbindungen, in einem stöchiometrischen Verhältnis von 0,8 bis 1,2 vorzugsweise 0,95 bis 1,1 bezogen auf die D-Pantothensäure zusetzt.

7. Verfahren zur Herstellung von D-Pantothensäure und/oder deren Natrium-, Kalium-, Ammonium-, Magnesium- oder Calcium-Salze enthaltenden Futtermittel-Additiven gemäß den Ansprüchen 5 bis 6,
**dadurch gekennzeichnet,**
**daß** man
a) aus einer unter Verwendung der gewünschten Hydroxyverbindungen gewonnenen D-Pantothenathaltigen Fermentationsbrühe gegebenenfalls vollständig oder teilweise die Biomasse und/oder einen Teil der Inhaltsstoffe abtrennt,
b) das so erhaltene Gemisch vorzugsweise durch Entfernen von Wasser aufkonzentriert, und
c) das das entsprechende Pantothenat enthaltende Futtermitteladditiv durch Trocknen, Sprühtrocknen oder Sprühgranulation in fester Form gewinnt.

8. Verfahren zur Herstellung von Tierfuttermittel-Additiven mit einem Gehalt an D-Pantothensäure und/oder dessen Natrium-, Kalium-, Ammonium-, Magnesium- oder Calciumsalzen in dem Bereich von etwa 20 bis 80 Gew.-% (Trockenmasse) aus Fermentationsbrühen, **gekennzeichnet durch** die Schritte
a) bevorzugt Entfernen von Wasser aus der Fermentationsbrühe (Aufkonzentration),
b) gegebenenfalls Entfernen der während der Fermentation gebildeten Biomasse in einer Menge von 0 bis 100 %,
c) Zusatz von einer oder mehreren der genannten Verbindungen zu den gemäß a) und b) erhaltenen Fermentationsbrühen, wobei die Menge der zugesetzten Verbindungen so bemessen ist, daß deren Gesamkonzentration im Tierfuttermittel-Additiv im Bereich von etwa 20 bis 80 Gew.-% liegt, und
d) Trocknen der gemäß c) erhaltenen Fermentationsbrühe, um das Tierfuttermittel-Additiv in der gewünschten Pulver- oder Granulatform zu erhalten.

9. Verfahren gemäß Anspruch 8,
**dadurch gekennzeichnet,**
**daß** man die Fermentationsbrühe vor oder nach der bevorzugt durchgeführten Aufkonzentrierung mit einem Oxid oder Hydroxid der genannten Alkali- oder Erdalkalimetalle versetzt.

10. Verfahren gemäß den Ansprüchen 5 bis 8,
**dadurch gekennzeichnet,**
**daß** man in der Fermentation Pilze oder Hefen einsetzt.

11. Verfahren gemäß Anspruch 8,
**dadurch gekennzeichnet,**
**daß** man der gegebenenfalls aufkonzentrierten Fermentationsbrühe eine oder mehrere L-Aminosäuren, ausgewählt aus der Gruppe L-Lysin, L-Valin, L-Alanin, L-Threonin oder L-Tryptophan, zusetzt.

12. Verfahren gemäß den Ansprüchen 5 bis 8,
**dadurch gekennzeichnet,**
**daß** man in der Fermentation Bakterien der Gattung Corynebacterium einsetzt.

13. Verfahren gemäß den Ansprüchen 5 bis 8,
**dadurch gekennzeichnet,**
**daß** man in der Fermentation Bakterien der Familie Enterobacteriaceae einsetzt.

## Claims

1. Animal feedstuffs additives which are based on a fermentation broth and comprise D-pantothenic acid and salts thereof or salts thereof,
**characterized in that**
they comprise
a) D-pantothenic acid and/or one of its salts in an amount of 20 to 80 wt.% (dry weight)
b) the biomass, in an amount of 0 to 100 %, formed during the fermentation of microorganisms which produce D-pantothenic acid,
c) at least the predominant portion of the further constituents of the fermentation broth, and
d) are present in solid, finely divided or granulated, free-flowing form.

2. Animal feedstuffs additives according to claim 1,
**characterized in that**
they comprise one or more of the salts chosen from the group consisting of the sodium, potassium, ammonium, magnesium or calcium salts of D-pantothenic acid.

3. Animal feedstuffs additives according to claim 1,
**characterized in that**
they are present in a spray-dried or spray-granulated form.

4. Animal feedstuffs additives according to one or more of the preceding claims,
**characterized in that**
they additionally comprise one or more of the L-amino acids chosen from the group consisting of L-methionine, L-lysine, L-valine, L-alanine, L-threonine or L-tryptophan.

5. Process for the preparation of feedstuffs additives comprising D-pantothenic acid and/or one of its salts in an amount of 20 to 80 wt.% (dry weight),
**characterized in that**
a) from fermentation broth comprising D-pantothenic acid and/or salts thereof, all or some of the biomass and/or a portion of the further constituents are separated off,
b) the hydroxide or oxide of an alkaline earth metal or alkali metal is added to the solution or broth obtained in this way,
c) the mixture obtained in this way is dried or spray-dried, spray-granulated or granulated.

6. Process according to claim 5,
**characterized in that**
an oxide or hydroxide chosen from the group consisting of sodium, potassium, ammonium, magnesium or calcium compounds is added in a stoichiometric ratio of 0.8 to 1.2, preferably 0.95 to 1.1, based on the D-pantothenic acid.

7. Process according to claims 5 to 6 for the preparation of feedstuffs additives comprising D-pantothenic acid and/or sodium, potassium, ammonium, magnesium or calcium salts thereof,
**characterized in that**
a) from a fermentation broth comprising D-pantothenate and obtained using the desired hydroxy compounds, optionally all or some of the biomass and/or a portion of the constituents is separated off,
b) the mixture obtained in this way is preferably concentrated by removal of water, and
c) the feedstuffs additive comprising the corresponding pantothenate is obtained in a solid form by drying, spray-drying or spray-granulation.

8. Process for the preparation of animal feedstuffs additives with a content of D-pantothenic acid and/or sodium, potassium, ammonium, magnesium or calcium salts thereof in the range from about 20 to 80 wt.% (dry weight) from fermentation broths, **characterized by** the steps
a) preferably removal of water from the fermentation broth (concentration),
b) optionally removal, in an amount of 0 to 100 %, of the biomass formed during the fermentation,
c) addition of one or more of the compounds mentioned to the fermentation broths obtained according to
a) and b), the amount of the compounds added being such that the total concentration thereof in the animal feedstuffs additive is in the range from about 20 to 80 wt.%, and
d) drying of the fermentation broth obtained according to c) in order to obtain the animal feedstuffs additive in the desired powder or granule form.

9. Process according to claim 8,
**characterized in that**
an oxide or hydroxide of the alkali metals or alkaline earth metals mentioned is added to the fermentation broth before or after the concentration which is preferably carried out.

10. Process according to claims 5 to 8,
**characterized in that**
fungi or yeasts are employed in the fermentation.

11. Process according to claim 8,
**characterized in that**
one or more L-amino acids chosen from the group consisting of L-lysine, L-valine, L-alanine, L-threonine or L-tryptophan are added to the optionally concentrated fermentation broth.

12. Process according to claims 5 to 8,
**characterized in that**
bacteria of the genus Corynebacterium are employed in the fermentation.

13. Process according to claims 5 to 8,
**characterized in that**
bacteria of the Enterobacteriaceae family are employed in the fermentation.

## Revendications

1. Acide D-pantothénique ou ses sels ou des additifs alimentaires pour animaux contenant ses sels à base de bouillon de fermentation, **caractérisés en ce qu'**ils
a) contiennent de l'acide D-pantothénique et/ou un de ses sels en une quantité de 20 à 80 % en poids (masse sèche),
b) contiennent la biomasse formée pendant la fermentation des microorganismes produisant l'acide D-pantothénique en une quantité de 0 à 100 %,
c) contiennent au moins la partie principale des autres constituants du bouillon de fermentation et
d) se trouvent sous une forme pouvant s'écouler, solide, finement divisée ou granulée.

2. Additifs alimentaires pour animaux selon la revendication 1, **caractérisés en ce qu'**ils contiennent un ou plusieurs des sels de l'acide D-pantothénique choisis parmi le groupe des sels de sodium, de potassium, d'ammonium, de magnésium ou de calcium.

3. Additifs alimentaires pour animaux selon la revendication 1, **caractérisés en ce qu'**ils se trouvent sous forme séchée par pulvérisation ou granulée par pulvérisation.

4. Additifs alimentaires pour animaux selon l'une ou plusieurs des revendications précédentes, **caractérisés en ce qu'**ils contiennent en outre un ou plusieurs des acides L-aminés, choisis parmi le group de la L-méthionine, la L-lysine, la L-valine, la L-alanine, la L-thréonine ou le L-tryptophane.

5. Procédé de production d'un additif alimentaire pour animaux contenant de l'acide D-pantothénique et/ou un de ses sels en une quantité de 20 à 80% en poids (masse sèche) à partir de bouillons de fermentation, caractérisé
a) en ce qu'on sépare le cas échéant complètement ou partiellement la biomasse et/ou une partie des autres constituants des bouillons de culture contenant de l'acide D-pantothénique et/ou ses sels,
b) en ce qu'on mélange la solution ou le bouillon ainsi obtenu avec l'hydroxyde ou l'oxyde d'un métal alcalino-terreux ou alcalin et
c) en ce qu'on sèche par pulvérisation, granule par pulvérisation ou granule le mélange ainsi obtenu.

6. Procédé selon la revendication 5, **caractérisé en ce qu'**on ajoute un oxyde ou un hydroxyde, choisi parmi le groupe des composés du sodium, du potassium, de l'ammonium, du magnésium ou du calcium dans un rapport stoechiométrique de 0,8 à 1,2, de préférence de 0,95 à 1,1, par rapport à l'acide D-pantothénique.

7. Procédé de production d'un additif alimentaire pour animaux contenant de l'acide D-pantothénique et/ou ses sels de sodium, de potassium, d'ammonium, de magnésium ou de calcium selon les revendications 5 à 6, caractérisé
a) en ce qu'on sépare le cas échéant complètement ou partiellement la biomasse et/ou une partie des constituants d'un bouillon de fermentation contenant du D-pantothénate obtenu en utilisant les composés hydroxy souhaités,
b) en ce qu'on concentre le mélange ainsi obtenu de préférence par élimination d'eau et
c) en ce que l'additif alimentaire pour animaux contenant le pantothénate correspondant est obtenu sous forme solide par séchage, séchage par pulvérisation ou granulation par pulvérisation.

8. Procédé de production d'additifs alimentaires pour animaux présentant une teneur en acide D-pantothénique et/ou en ses sels de sodium, de potassium, d'ammonium, de magnésium ou de calcium dans la plage d'environ 20 à 80% en poids (masse sèche) à partir de bouillons de fermentation, **caractérisé par** les étapes
a) de préférence d'élimination d'eau du bouillon de fermentation (concentration),
b) le cas échéant d'élimination de la biomasse formée pendant la fermentation en une quantité de 0 à 100 %,
c) d'addition d'un ou de plusieurs des composés mentionnés aux bouillons de culture obtenus selon a) et b), la quantité des composés ajoutés étant mesurée de telle manière que leur concentration totale dans l'additif alimentaire pour animaux se situe dans la plage d'environ 20 à 80 % en poids et
d) de séchage du bouillon de fermentation obtenu selon c), afin d'obtenir l'additif alimentaire pour animaux sous la forme souhaitée de poudre ou de granulat.

9. Procédé selon la revendication 8, **caractérisé en ce qu'**on additionne le bouillon de fermentation, avant ou après la concentration réalisée de préférence, d'un oxyde ou d'un hydroxyde des métaux alcalins ou alcalino-terreux mentionnés.

10. Procédé selon la revendication 5 à 8, **caractérisé en ce qu'**on utilise dans la fermentation des champignons ou des levures.

11. Procédé selon la revendication 8, **caractérisé en ce qu'**on ajoute au bouillon de fermentation le cas échéant concentré un ou plusieurs acides L-aminés, choisis parmi le groupe de la L-lysine, la L-valine, la L-alanine, la L-thréonine ou le L-tryptophane.

12. Procédé selon les revendications 5 à 8, **caractérisé en ce qu'**on utilise dans la fermentation des bactéries du type Corynebacterium.

13. Procédé selon les revendications 5 à 8, **caractérisé en ce qu'**on utilise dans la fermentation des bactéries de la famille des Enterobacteriaceae.
